# EUROPEAN PATENT SPECIFICATION

(11) **EP 3 167 051 B1**
(45) Date of publication and mention of the grant of the patent: **04.08.2021**
(21) Application number: 15734588.5
(22) Date of filing: 18.06.2015
(51) Int. Cl.: C07K 14/415, C12N 9/02, C12N 15/82

(54) **PHYTOPHTHORA RESISTANT PLANTS BELONGING TO THE SOLANACEAE FAMILY**
PHYTOPHTORA RESISTENTE PFLANZEN DER FAMILIE SOLANACEAE
PLANTES RÉSISTANTES À PHYTOPHTHORA APPARTENANT À LA FAMILLE DES SOLANACEAE

(30) Priority: 18.06.2014 WO PCT/EP2014/062802
(43) Date of publication of application: 17.05.2017
(73) Proprietor: Enza Zaden Beheer B.V., 1602 DB Enkhuizen (NL)
(72) Inventor: VAN SCHIE, Christianus Cornelis Nicolaas, 1025 WH Amsterdam (NL); POSTHUMA, Karin Ingeborg, 1601 AJ Enkhuizen (NL); ZEILMAKER, Tieme, 3823 JJ Amersfoort (NL); DE BOER, Geert Johannes, 1974 LV IJmuiden (NL)
(74) Representative: van Kooij, Adriaan
(86) International application number: PCT/EP2015/063682
(87) International publication number: WO 2015/193418

(56) References cited:
- EP-A1- 2 455 473
- WO-A1-2008/092505
- WO-A1-2015/029031
- WO-A1-2015/106796
- Tieme Zeilmaker: "Functional and applied aspects of the DOWNY MILDEW RESISTANT 1 and 6 genes in Arabidopsis (PhD thesis)", , 6 February 2012 (2012-02-06), pages FP-147, XP002730064, Universiteit Utrecht Retrieved from the Internet: URL:http://web.science.uu.nl/pmi/publicati ons/PDF/2012/Proefschrift-Zeilmaker-2012.p df [retrieved on 2014-09-23]
- DATABASE UniProt [Online] 3 April 2013 (2013-04-03), "SubName: Full=Uncharacterized protein;", XP002730065, retrieved from EBI accession no. UNIPROT:M0ZIQ1 Database accession no. M0ZIQ1 & XU XUN ET AL: "Genome sequence and analysis of the tuber crop potato", NATURE (LONDON), vol. 475, no. 7355, July 2011 (2011-07), page 189, ISSN: 0028-0836(print)
- DATABASE UniProt [Online] 3 April 2013 (2013-04-03), "SubName: Full=Uncharacterized protein;", XP002730066, retrieved from EBI accession no. UNIPROT:M1CK41 Database accession no. M1CK41 & XU XUN ET AL: "Genome sequence and analysis of the tuber crop potato", NATURE (LONDON), vol. 475, no. 7355, July 2011 (2011-07), page 189, ISSN: 0028-0836(print)
- DATABASE UniProt [Online] 28 November 2012 (2012-11-28), "SubName: Full=Uncharacterized protein;", XP002730067, retrieved from EBI accession no. UNIPROT:K4C928 Database accession no. K4C928 & BUDIMAN MUHAMMAD A ET AL: "A deep-coverage tomato BAC library and prospects toward development of an STC framework for genome sequencing", GENOME RESEARCH, vol. 10, no. 1, January 2000 (2000-01), pages 129-136, ISSN: 1088-9051
- TIEME ZEILMAKER ET AL: "DOWNY MILDEW RESISTANT 6 and DMR6-LIKE OXYGENASE 1 are partially redundant but distinct suppressors of immunity in Arabidopsis", THE PLANT JOURNAL, vol. 81, no. 2, 9 December 2014 (2014-12-09), pages 210-222, XP055209378, ISSN: 0960-7412, DOI: 10.1111/tpj.12719
- None

## Description

The present invention relates to *Phytophthora* resistance sweet pepper or *Capsicum spp.* plants belonging to *Solanaceae* family wherein said resistance is encoded by a combination of two separate genes.

The plant pathogen *Phytophthora* (class: *Oomycetes,* order: *Peronosporales,* family: *Pythiaceae*) is a genus of plant-damaging Oomycetes (water molds) capable of causing large economic losses on crops worldwide, as well as environmental damage in natural ecosystems. *Phytophthora* pathogens are mostly pathogens of dicotyledons and generally are host-specific parasites. The genus was first described by Heinrich Anton de Bary in 1875. Approximately 100 species have been described amongst which economically important plant pathogens as *Phytophthora capsici, Phytophthora infestans,* and *Phytophthora nicotinae.*

*Phytophthora infestans* was the infective agent of the potato blight that caused the Great Irish Famine (1845-1849), and still remains the most destructive pathogen of solanaceous crops, including tomato and potato. The soya bean root and stem rot agent, *Phytophthora sojae,* has also caused longstanding problems for the agricultural industry. In general, plant diseases caused by this genus are difficult to control chemically, and thus the growth of resistant cultivars is the main management strategy. Other important Phytophthora diseases are:
*Phytophthora cactorum* - causes rhododendron root rot affecting rhododendrons, azaleas and causes bleeding canker in hardwood trees; *Phytophthora capsici* - infects Cucurbitaceae fruits, such as cucumbers and squash, *Phytophthora cinnamomi* - causes cinnamon root rot affecting woody ornamentals including arborvitae and azalea, *Phytophthora fragariae* - causes red root rot affecting strawberries; *Phytophthora kernoviae* - pathogen of beech and rhododendron, also occurring on other trees and shrubs including oak, and holm oak, *Phytophthora megakarya* - one of the cocoa black pod disease species, is invasive and probably responsible for the greatest cocoa crop loss in Africa; *Phytophthora palmivora* - causes fruit rot in coconuts and betel nuts, *Phytophthora ramorum*, *Phytophthora quercina* - causes oak death, and *Phytophthora sojae* - causes soybean root rot.

*Phytophthora* is sometimes referred to as a fungal-like organism but it is classified under a different kingdom: *Chromalveolata* (formerly *Stramenopila* and previously *Chromista*). *Phytophthora* is morphologically very similar to "true" fungi yet its evolutionary history is quite distinct. In contrast to fungi, chromalveolatas are more closely related to plants than animals. Whereas fungal cell walls are made primarily of chitin, chromalveolata cell walls are constructed mostly of cellulose. Ploidy levels are different between these two groups; *Phytophthora* have diploid (paired) chromosomes in the vegetative (growing, non-reproductive) stage of life, Fungi are almost always haploid in this state. Biochemical pathways also differ, notably the highly conserved.

*Phytophthoras* may reproduce sexually or asexually. In many species, sexual structures have never been observed, or have only been observed in laboratory matings. In homothallic species, sexual structures occur in single culture. Heterothallic species have mating strains, designated as A1 and A2. When mated, antheridia introduce gametes into oogonia, either by the oogonium passing through the antheridium (amphigyny) or by the antheridium attaching to the proximal (lower) half of the oogonium (paragyny), and the union producing oospores. Like animals, but not like most "true" fungi, meiosis is gametic, and somatic nuclei are diploid. Asexual (mitotic) spore types are chlamydospores, and sporangia which produce zoospores. Chlamydospores are usually spherical and pigmented, and may have a thickened cell wall to aid in its role as a survival structure. Sporangia may be retained by the subtending hyphae (non-caducous) or be shed readily by wind or water tension (caducous) acting as dispersal structures. Also, sporangia may release zoospores, which have two unlike flagella which they use to swim towards a host plant.

The *Solanaceae,* or nightshades, is an economically important family of flowering plants. The family ranges from herbs to trees, and includes a number of important agricultural crops, medicinal plants, spices, weeds, and ornamentals. Many members of the family contain potent alkaloids, and some are highly toxic.

The *Solanaceae* family belongs to the order *Solanales,* in the asterid group dicotyledons (*Magnoliopsida*). The *Solanaceae* family consists of approximately 98 genera and some 2,700 species, with a great diversity of habitats, morphology and ecology.

The family has a worldwide distribution being present on all continents except Antarctica. The greatest diversity in species is found in South America and Central America. *Solanaceae* includes a number of commonly collected or cultivated species. Perhaps the most economically important genus of the family is *Solanum,* which comprises potato (S*olanum tuberosum,* in fact, another common name of the family is the "potato family"), tomato (S*olanum lycopersicum*), and the aubergine or eggplant (S*olanum melongena*). Another important genus is *Capsicum* comprising peppers and sweet or bell peppers *(Capsicum annuum).*

The genus *Physalis* produces the so-called groundcherries, as well as the tomatillo (*Physalis philadelphica*), the Cape gooseberry and the Chinese lantern. The genus *Lycium* contains the boxthorns and the wolfberry *Lycium barbarum. Nicotiana* contains, among other species, the tabacco plant. Some other important members of *Solanaceae* include a number of ornamental plants such as Petunia, Browallia and Lycianthes, the source of psychoactive alkaloids, Datura, Mandragora (mandrake), and Atropa belladonna (deadly nightshade). Certain species are universally known for their medicinal uses, their psychotropic effects or for being poisonous.

With the exception of tobacco (*Nicotianoideae*) and petunia (*Petunioideae*), most of the economically important genera are contained in the subfamily *Solanoideae.* Finally, but not less importantly, the *Solanacea* include many model organisms which are important in the investigation of fundamental biological questions at a cellular, molecular and genetic level, such as tobacco, petunia and *N. benthaminia.*

WO 2008/092505 discloses plants, which plants are resistant to a pathogen of viral, bacterial, fungal or oomycete origin, wherein the plants have a reduced level, reduced activity or complete absence of DMR6 protein as compared to the plants which are not resistant to the pathogen, in particular organisms of the Fungi or the phylum Oomycota.

Tieme Zeilmaker: "Functional and applied aspects of the DOWNY MILDEW RESISTANT 1 and 6 genes in Arabidopsis (PhD thesis)", 6 February 2012 (2012-02-06), Universiteit Utrecht discloses various DMR6 homologs having partially redundant but distinct functions in supressing immunity in *Arabidopsis.*

Considering the economic importance of many plant members of the *Solanaceae* family and the destructive effect of the plant pathogen *Phytophthora* on many members of this family, it is an object, amongst other objects, of the present invention to provide *Phytophthora* resistant plants and especially sweet pepper *(Capsicum annuum)* or *Capsicum* spp.

The above object, amongst other objects, is met by the present invention by providing plants as outlined in the appended claims.

Disclosed are plants belonging to the *Solanaceae* family wherein the present plants comprise a genetic trait providing *Phytophthora* resistance and wherein the present resistance trait is encoded by a combination of at least two genes having a reduced expression, or reduced transcription, or a reduced activity of proteins encoded by the present genes as compared to the same plant belonging to *Solanaceae* family being susceptible to *Phytophthora.*

Disclosed are plants belonging to the *Solanaceae* family selected from the group consisting of potato, petunia, tomato, aubergine, eggplant, tobacco and sweet pepper.

According to the present invention, the present plants are sweet pepper (*Capsicum annuum*) or *Capsicum* spp., the *Phytophthora* resistance is resistance to *Phytophthora capsici* and the combination of at least two genes are genes encoding proteins according to SEQ ID No. 16 and SEQ ID No. 17 or proteins having at least 90% sequence identity with SEQ ID No. 16 and/or SEQ ID No. 17, preferably at least 95% sequence identity, most preferably at least 99% sequence identity.

Disclosed are plants belonging to the *Solanaceae* family wherein the plants comprise a genetic trait providing *Phytophthora* resistance, wherein the present resistance trait is obtainable by down regulating the activity of combination of two genes or reducing the activity of proteins encoded by the genes in a *Phytophthora* susceptible plant, wherein the two genes encode the combinations of SEQ ID Nos. 1 and 2 or SEQ ID Nos. 3 and 4 or SEQ ID Nos. 5 and 6 or proteins having at least 80%, 85%, or 90% sequence identity therewith such as 91%, 92%, 93% and 94% sequence identity, preferably at least 95% sequence identity, such as 96%, 97%, 98% and 99% sequence identity.

Given the advantageous properties of the present genes for providing *Phytophthora* resistance plants, disclosed is the use of a combination of two genes, wherein said combination of two genes encode protein combinations are SEQ ID Nos. 16 and 17 or proteins having at least 90% sequence identity for providing *Phytophthora* resistance in is sweet pepper (*Capsicum annuum*) or *Capsicum* spp plants.

The disclosed use comprises a reduced expression, or reduced transcription, of the present genes or a reduced activity of proteins encoded by the present genes as compared to sweet pepper (*Capsicum annuum*) or *Capsicum* spp plants being susceptible to *Phytophthora.*

Disclosed are proteins and genes suitable for providing *Phytophthora* resistance to plants wherein the proteins are selected from the group consisting of SEQ ID No. 1, 2, 3, 4, 5, 6, 16, 17, 18 and 19 and proteins having at least 90% sequence identity therewith, preferably at least 95%, most preferably at least 99%.

The invention will be further illustrated in the examples below.

### Examples

### Example 1 (potato)

### RNAi constructs targeting potato SEQ ID NOS. 7 and 8

3 different RNAi constructs were made, harboring/targeting:
1. 5' end of SEQ ID NO 7: equivalent to coding sequence -159-200 (-159 from start means in 5'utr).
2. Chimera of 5' end of SEQ ID NOS. 7 and 8: equivalent to coding sequence 4-199 + 1-204.
3. Middle part of SEQ ID NO. 7 (highly homologous to middle of SEQ ID NO 8): equivalent to coding sequence 334-743.

The fragments were amplified from genomic DNA and cloned into the pENTR-D-TOPO vector. For the chimeric construct, 2 fragments were coupled using primers with complementary overhangs, and subsequent extension and amplification to create the fused fragment. Fragments were transferred using a Gateway LR reaction to the RNAi vector pK7GWiWG2 (Karimi et al., 2002, Trends Plant Sci 7), creating an inverted repeat with hairpin structure. Because the pK7GWiWG2 vector requires Streptomycin for bacterial selection, and the Agrobacterium strain used for potato transformation (LBA4404) already carries a Streptomycin selection marker, the complete RNAi (hairpin) cassette was transferred to a different plant transformation vector, pGreen0029 (bacterial as well as plant selection marker = Kanamycin) (Hellens et al., 2000, Plant Mol Biol 42). The final constructs allow stable expression of a 35S-promoter driven hairpin RNA that forms a silencing-inducing dsRNA, after the hairpin-loop forming intron gets spliced out. At least six independent T1 transformants were maintained for each construct.

### Phytophthora infestans Assay details

Detached leaves were taken from T1 (first generation transgenics) plants, and placed in a tray with 100% RH with petioles in wet cotton-wool or Oasis. *Phytophthora infestans* (P.inf) zoospores/sporangia were harvested from P.inf cultures (rye-sucrose-agar plates), and a 10ul drop of spore suspension containing 10e3 sporangia (10e5/ml) was placed on each side of the midvein. Trays were incubated at 18C. Leaf infection rates were scored on day 11, as 1. Completely infected/overgrown, 2. Partially infected (10-50% area), and 3. Clean (<10% area).

As shown in figures 1 and 2, the double silenced (SEQ ID NO. 7 & 8) plants of figure 2a show that only 50% is infected, the double silenced (chimeric) plants of figure 2b show that only 60% is infected, whereas the control group of figure 1 shows that all plants were infected. As shown in figure 3, 40 to 50% of the both SEQ ID NO. 7 and SEQ ID NO. 8 silenced plants are clean, whereas the plants having only SEQ ID NO. 7 silenced only 10% of the plants score partially infected. Accordingly, silencing of both SEQ ID NO. 7 and 8 provides resistance to *Phytophthora*
*infestans.*

### Example 2 (petunia)

Transposon insertion lines were identified from a collection/library (Vandenbussche et al., 2008, Plant Journal 54). 2 dTph1 transposon insertion alleles were found in SEQ ID NO 9 and 3 dTph1 transposon insertion alleles in SEQ ID NO 10. Several crosses were made to generate double mutants.

### Phytophthora nicotianae Assay details

Plants were grown in standard potting soil, individually potted, at 23C.
*P.nicotianae* spores were harvested from cultures (lima-bean-agar or V8-agar plates), and 2ml of spore suspension containing 10e4 (assay Sept) spores was dripped onto the soil with each plant. Plant collapse was monitored regularly.

As shown in figure 4, double mutants, i.e. plants having mutations in both SEQ ID NO 9 and SEQ ID NO 10 have a percentage of living plants of 45%, whereas the percentage of living plants of single mutants (mutant in SEQ ID NO. 9 or SEQ ID NO. 10) is only 20%.

### Example 3 (tomato)

Tomato plants were transformed with two constructs, either for providing over expression of both SEQ ID NO. 11 and 12, or for providing silencing of both SEQ ID NO. 11 and 12. Tomato SEQ ID NO. 11 silencing constructs were generated using Gateway cloning of a 300 bp fragment identical to the middle part of the CDS of SEQ ID NO. 11.

### Sequence:

### Using primers:

| | |
|---|---|
| S. Lycopersicum AttB1-F | aaaaagcaggcttcttgggtgaacaaggacaaca |
| S.Lycopersicum AttB2-R | agaaagctgggtaaaacgaagccactgacatcc |

The generated ENTRY vector was Gateway cloned into the pHellsgate12 binary vector. Following Agrobacterium transformation according standard procedure for tomato. The silencing constructs were able to silence both SEQ ID NO. 11 and 12, due to similarities in the sequences.

Offspring from transformed tomato plants were subjected to a disease test by inoculation of *Phytophthora infestans* isolate US11. 7 days after inoculation the plants were visually analysed by scoring leaves on a visual scale from 1 to 9, wherein 1 means susceptible and 9 means resistant. As a control for susceptible the plants TS33, TS19 and OT9 were used. As control for resistant the known resistant wild accession LA1269 is used. Per plant 8 leaves were measured. Table below provides the average score from the 8 leaves per plant.

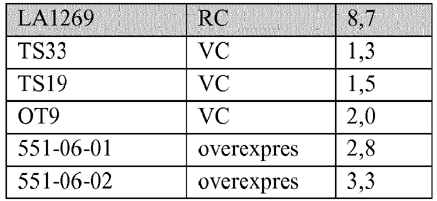

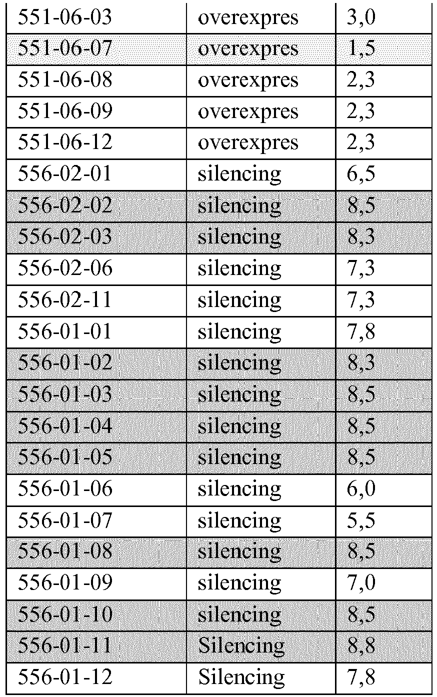

In the table is shown that the SEQ ID NO. 11 and 12overexpressing plants are susceptible for isolate US11. The silenced plant provides significant higher scores than the susceptible control OT9. For example plant 556-01-08 has an average score of 8.5. A sample of this plant is shown in figure 5 in box G10, and is not infected similar to resistant control plant LA1296 as shown in box D8. Accordingly, silencing of both SEQ ID NO. 11 and 12 provides resistance to *Phytophthora infestans.*

### Example 4 (N. benthamiana)

To investigate if *DMR6* mediated resistance in *N. benthamiana* against *Phytophthora infestans* the *DMR6* ortholog was cloned in an VIGS expression vector. *N. benthamiana* plants were silenced for *DMR6* and subsequent dropinoculated with a spore solution of *P. infestans.* Up to 7 dpi, plants were scored for disease development. This experiment was performed three times with similar results. *DMR6* silencing clearly results in less colonization by the pathogen compared to the empty vector control or wildtype non silenced plants.

### Sequence of silencing construct DMR6:

VIGS was used to silence and test the function of *N. benthamiana* DMR6. As a visual control for the procedures, the PDS gene was also targeted. The PDS silenced plants were used to indicate at which time the silencing started. The silencing must have started ± 2 days before the bleaching is observed due to the protein levels already established in the plant. But at the start of the silencing time frame, the silencing will not be at a high rate and waiting two days gives the mechanism time to establish a good silencing rate. Another advantage of using PDS silenced plants is that they give an indication which parts of the plants are silenced. The bleaching was observed in all parts above the inoculation. In the vacuum inoculated plants the new tissue showed strong photobleaching and parts of some of the cotelydons showed white areas as well. In the syringe inoculated plants the bleaching was the strongest in the youngest leaves, in full grown leaves the pigment is already synthesized and silencing will be harder to observe visually. Bleaching of the PDS silenced plants was observed 8-10 days past inoculation with the Agrobacterium strain containing the TRV vector. On day 8 the infection assays were started.

Two separate VIGS experiments were performed. Plants from both experiments were used for detached leaf infection assays. The P. capsici infection assay was done 8 days after Agro-infiltration, on leaves 2-6 above infiltrated leaf. Zoospore solution containing 5^{∗}10³ spores/ml-1 was applied to detached leaves. The first VIGS showed low infection rates of the *P. capsici* after 7 days (see table 1.). Ideally, 100% of non-silenced leaves show infection.

**Table 1. Results from the infection assay of the 1^{st} VIGS expteriment. No DMR6 silenced leaves showed infection. 3 out of 7 Non-silenced leaves showed infection.**

| | Infected | Not infected | Total | % Infected |
|---|---|---|---|---|
| DMR6 silenced | 0 | 11 | 11 | 0% |
| Not silenced | 3 | 4 | 7 | 43% |

This low sporulation is thought to be the cause of the low infection of the *N. benthamiana* leaves. However, none of the DMR6 silenced leaves showed infection symptoms, as opposed to 40% of the leaves which were not silenced showed infection. To increase infection rate, changes were made in the acquiring of spores and infection method causing more aggressive infection during the second infection assay. One DMR6 silenced uninfected leaf was found and other DMR6 silenced leaves showed delayed infection and slower spreading of infection.

### Example 5

### Introduction

A number of genes encoding negative regulators of immunity are activated during pathogen infection so that the inducible defense response is controlled and down-regulated to prevent over-activation. Examples are the Nudix hydrolase-encoding *NUDT7* and transcription factor-encoding *WRKY48* that are induced upon infection or MAMP treatment. Similarly, the *DOWNY MILDEW RESISTANT 6* gene (*DMR6*) is activated during infection with compatible and incompatible isolates of the downy mildew *Hyaloperonospora arabidopsidis.* Inactivation of *DMR6* by mutation leads to a low constitutive activation of defense-related genes and resistance to the downy mildew *H. arabidopsidis.*

DMR6 belongs to the superfamily of 2-oxoglutarate Fe(II) dependent oxygenases (2OG oxygenases, Pfam domain PF03171). This superfamily comprises 151 members in Arabidopsis. However, for most of these proteins, including DMR6, their metabolic activity is unknown. 2OG oxygenases are known to catalyze a plethora of reactions that involve the oxidation of a substrate using molecular O₂. They commonly use iron as co-factor and require 2-oxoglutarate as co-substrate for supplying two electrons. A general hallmark of these enzymes is the presence of the conserved HxD/ExₙH motif located on a double-stranded beta sheet. Together with two four-stranded beta sheets (jelly roll fold) it capsulates the active center. 2OG oxygenases are implicated in secondary metabolism and biosynthesis of signaling molecules e.g. the biosynthesis of flavonoids, gibberellins, and alkaloids.

In this example, we functionally analyse the Arabidopsis DMR6 oxygenase and related *DMR6-LIKE OXYGENASES* (DLO) 1 and 2. Overexpression of *DMR6, DLO1,* and *DLO2* increased disease susceptibility indicating the three proteins can act as a negative regulator of immunity. *DLO1,* but not *DLO2,* is highly co-regulated with *DMR6,* however they differ in their spatial expression during downy mildew-infection. The *dmr6-3_dlo1* double mutant was found to be completely resistant to *H. arabidopsidis* and showed a strongly reduced growth associated with high levels of salicylic acid.

### Results

### Overexpression of DMR6 results in enhanced susceptibility to (hemi-)biotrophic pathogens

Seedlings of the *dmr6-1* mutant were previously described to be more resistant to *H. arabidopsidis,* but not to *P. syringae.* When tested on adult *dmr6-1* plants, however, strong resistance to *P. syringae* DC3000 was observed. Also adult *dmr6-1* plants were more resistant to the obligate biotroph *H. arabidopsis* compared to seedlings. In addition, strong resistance to the hemi-biotrophic oomycete *Phytophthora capsici* was evident in *dmr6-1* plants when compared to the parental line L*er eds1-2*; whereas all *dmr6-1* mutant plants survived, *P. capsici* destroyed the vast majority of plants of the parental line and complemented *dmr6-1* mutant. The resistance of the *dmr6-1* mutant to different (hemi-)biotrophs suggests that in wild-type plants DMR6 negatively regulates immunity to these pathogens.

To study this, the *DMR6* coding sequence was expressed from the constitutive 35S promoter in transgenic Col-0 lines. The *DMR6-*overexpression lines showed a clear increase in disease susceptibility to *H. arabidopsidis* and *P. syringae.* The level of *H. arabidopsidis* sporulation, which is a measure of downy mildew infection, was doubled in *DMR6-*overexpression lines compared to the control. Also the development of disease-associated chlorosis was more pronounced in *DMR6-*overexpression lines than in non-transgenic Col-0 plants.

The increased susceptibility of six-week old plants to *P. syringae* bacteria was also clearly visible. While the control line (Col-0) showed a relatively low level of chlorosis and lesions at 3 days post inoculation, the *DMR6-*overexpression line showed more severe disease symptoms, i.e. more chlorosis and more and larger lesions. The increased susceptibility of *DMR6-*overexpressors to *P. syringae* infection was confirmed by bacterial growth assays that showed increased bacterial titres at 1 and 3 days post inoculation compared to the Col-0 control. Furthermore, expression of the defense marker genes PR-1, PR-2, and PR-5 in uninfected leaf tissue was reduced by 50 to 80% in the *DMR6-*overexpression line compared to wild type Col-0 plants that already have a very low level of expression. The reduced immunity of the *DMR6-*overexpression line, together with the enhanced resistance of the *drm6-1* mutant, strongly supports the role of DMR6 as a negative regulator of immunity.

### DMR6 and DMR6-LIKE OXYGENASEs represent separate branches of a distinct clade in flowering plants

The Arabidopsis genome contains more than 150 2OG oxygenase genes some of which are similar to DMR6. To analyse the evolutionary conservation of DMR6 and related oxygenases in flowering plants we phylogenetically analysed the family of 2OG oxygenases that contain the 20G-Fe(II) oxygenase superfamily Pfam domain PF03171. From *Arabidopsis thaliana* and eighteen flowering plants, of which genome sequences and protein models were available in the Phytozome v7.0 database (www.Phytozome.net), a total of 2951 proteins containing the PF03171 domain were selected using the HMMER3 algorithm. To filter small protein fragments and remove very large proteins, we only included proteins that did not exceed a 20% length difference to DMR6. Furthermore, only proteins were retained that have a length difference of the oxidoreductase domain of less than 20% compared to DMR6. This resulted in a selection of 2038 proteins that fulfil all criteria, including 110 of 151 predicted *A. thaliana* 2OG oxygenases. Phylogenetic clustering resulted in a tree in which many distinct clades representing different enzyme activities are shown. Well-characterized oxygenases include flavonone-3-hydroxylase (F3H), 1-aminocyclopropane-1-carboxylic acid (ACC) oxidase, and anthocyanidin synthase (ANS) which are present in distinct clades different from the DMR6 clade. Two separate branches can be distinguished in the DMR6 clade that each contain 2OG oxygenases from dicots and monocots indicating that these subclades were already present in the ancestor of all flowering plants or earlier (82% bootstrap confidence). Gene duplications in the DMR6 clade are frequent in monocots in the upper part of the tree and in soybean and grapevine in both branches of the DMR6 clade. In the lower subclade, two *A. thaliana* DMR6 homologues cluster together with two proteins from *Arahidopsis lyrata* suggesting they are the result of a relatively recent gene duplication in the common ancestor of these two species.

These *A. thaliana* proteins are now designated DMR6-LIKE OXYGENASE or DLO (with gene At4g10500 encoding DLO1 and At4g10490 encoding DLO2). Also the DLO subclade shows a clear separation of the monocot and dicot proteins suggesting that the ancestor of all flowering plants already possessed a DLO besides DMR6. Grouping closely to DMR6, the DLOs form an interesting group that was subsequently analysed in more detail, focusing on the *A. thaliana DLO1* and *DLO2* genes.

### Overexpression of DLO1 and DLO2 complements the dmr6 mutant

The DLOs could have the same biological activity as DMR6 and were, therefore, tested for complementation of the *dmr6-1* mutant. To this end, *DLO1* and *DLO2* were expressed under the constitutive 35S promoter and transformed into the *dmr6-1* mutant background. Four independent T3 lines, transformed with 35S:*DLO1* or 35S:*DLO2,* were analysed for their expression level and 3 lines per construct were selected that showed clear transgene expression. To check for complementation, 2-week old plants were infected with *H. arabidopsidis* isolate Cala2 and at 5 days post inoculation (dpi) the number of spores per mg seedlings was scored as measure of susceptibility.

Intriguingly, while *dmr6-1* showed clear resistance, the 35S::*DLO1* and 35S::*DLO2* plants were highly susceptible, similar to or higher than L*er eds1-2* that is the parental line of the *dmr6-1* mutant. As both *DLO1* and *DLO2* can complement the *dmr6-1* mutant phenotype we conclude that the DLOs have a function similar to that of DMR6.

As overexpression of *DMR6* in the Col-0 background results in enhanced susceptibility to downy mildew and other pathogens, we next investigated if overexpressing *DLO1* and *DLO2* would also make Col-0 more susceptible. Transformants expressing the 35S:*DLO1* and 35S:*DLO2* transgenes were selected and as controls Col-0 overexpressing *DMR6* and the highly susceptible Col *eds1-2* mutant were included. Disease assays with *H. arabidopsidis* showed that overexpression of *DLO1* and *DLO2* enhanced susceptibility compared to the Col-0 parental line as shown by the higher level of sporulation. The observed enhanced susceptibility was comparable to the *DMR6* overexpression plants and the Col *eds1-2* mutant. This confirms that the DLO1 and DLO2 protein have an activity similar or identical to DMR6 resulting in the same phenotypic effects.

### Expression of DLO1, but not DLO2, is immunity-related

*The DLO1 and DLO2 complementation and overexpression lines were all generated using the 35S promoter. It is, however, likely that the expression of the wild-type DLOs is highly regulated similar to that of DMR6, which is strongly activated during plant defense. Therefore, we analysed publicly available gene expression data to determine if DLO1 and DLO2 show immunity-related expression similar to DMR6. For this analysis, data of* 9 different Affymetrix microarray experiments all dealing with transcriptional profiling after pathogen attack, defense related hormone application and elicitor/effector treatment, were used.

The expression analysis was focused on 30 2OG oxygenases that belong to the large clade containing the DLOs and DMR6. Hierarchical clustering of the genes allowed grouping of the 2OG oxygenase genes according to their expression patterns, providing information about which genes are co-regulated during plant immune responses. Strikingly, DLO1 clusters with DMR6, whereas DLO2 does not show any co-regulation with DMR6 or DLO1. DMR6 and DLO1 are both activated after infection with the downy mildew *H. arabidopsidis,* the powdery mildew *Erysiphe orontii,* and the bacterium *P. syringae* and as well as SA treatment. DLO2 clusters well away from DMR6 and DLO1 and appears to be unresponsive in the different experiments. Further analysis of available microarray data using Genevestigator revealed that *DLO2* is not expressed in response to any treatment or in any tissue, except for siliques, suggesting that *DLO2* does not have a role in immunity of the vegetative plant tissues.

The responsiveness of the *DLOs* to *H. arabidopsidis* infection was experimentally verified by quantitative PCR (qPCR). *DMR6* and *DLO1* are highly activated in plants infected with a compatible or incompatible isolate of *H. arabidopsidis.* Also following treatment with the SA mimic BTH, both *DMR6* and *DLO1* are strongly activated. In contrast, *DMR6* and *DLO1* are unresponsive to methyl jasmonate (MeJA), which is known to activate jasmonic acid-induced genes. *DLO2* expression is undetectable (cT values higher than 35) in the different experimental conditions confirming the Genevestigator data. The fact that both *DMR6* and *DLO1* are activated during the plant's immune response suggests that in leaves of wild-type plants DLO1 also acts as a negative regulator of defense. However, the question remains why the *dmr6* mutants have such a clear resistance phenotype in the presence of an intact *DLO1* gene that could take over *DMR6* function?

### DLO1 and DMR6 show different spatial expression in infected leaves

To analyze the tissue-specific expression of *DLO1* during downy mildew infection, we generated transgenic lines containing a construct with the *DLO1* promoter fused to the *GUS* reporter gene (pro*_{DLO1}*:*GUS*). Since we did not observe any expression of *DLO2,* no GUS fusion with the promoter of *DLO2* was constructed. Following *H. arabidopsidis* infection, *DMR6* spatial expression was specifically detected to the sites that are in direct contact with the pathogen as has been described previously. In contrast, *DLO1* expression was not induced in cells that are in close contact with the pathogen but only in or around the main veins of infected cotyledons and leaves. Interestingly, *DLO1* expression was observed only in areas of the leaf that are close to *H. arabidopsidis* infection sites, indicating that the activation of *DLO1* depends on the presence of the pathogen. The absence of *DLO1* activity in haustoria-containing cells could explain why DLO1 cannot fully complement for loss of DMR6 activity in the *dmr6* mutants. Whereas these data show distinct activities of the *DMR6* and *DLO1* genes, the extent of redundancy of these genes is unclear and was therefore further studied genetically.

### DLO1 function is partially redundant with DMR6

Redundancy analysis in mutant lines is best performed in the same genetic background. We, therefore, obtained mutants in the Col-0 background for *DMR6* (GABI-KAT line GK-249H03.01, designated mutant *dmr6-3*) and *DLO1* (SALK line 059907, named *dlo1*)*. dmr6-3_dlo1* double mutants were generated and phenotypically analyzed together with the *dmr6-3* and *dlo1* single mutants, as well as with the parental Col-0 line. The level of susceptibility to *H. arabidopsidis* Waco9 was strongly reduced in the *dmr6-3* mutant, but only slightly reduced in the *dlo1* mutant. Combining the two mutations in the *dmr6-3_dlo1* double mutant resulted in plants that showed complete resistance to *H. arabidopsidis.*

We next tested the level of defense gene expression in the mutants, as previous research on the *dmr6-1* and *dmr6-2* mutants showed increased levels of expression of *PR-1* and other defense genes. Also the *dmr6-3* mutant showed elevated expression of *PR-1, PR-2* and *PR-5,* confirming previous results. The *dlo1* mutant only showed no significant induction of expression of the three PR genes.

In contrast, the *dmr6-3_dlo1* double mutant showed extremely high levels of defense gene expression. *PR-1* transcripts were more than 30,000-fold higher in more the *dmr6-3_dlo1* mutant than in Col-0, and a more than 100-fold higher than in the *dmr6-3* single mutant. In the tested mutants there was a clear correlation between the level of resistance to downy mildew and increase in defense gene expression, suggesting that resistance is caused by activation of plant immune responses. Our data shows that the *dlo1* mutation enhances the immunity of the *dmr6-3* single mutant, indicating DLO1 and DMR6 act partially redundant.

This was further corroborated by the growth phenotype of the mutants. Plants grown for 5,5 weeks under short day conditions showed striking differences between the genotypes. Whereas the *dlo1* mutant grows similar to Col-0, and the *dmr6-3* mutant only shows a slight growth reduction, the *dmr6-3_dlo1* double mutant displayed strong growth reduction resulting in dwarfed plants. The growth reduction and level of resistance to downy mildew are correlated in the tested mutants, suggesting these two phenotypes are functionally linked.

It is well known that strong activation of plant immunity can be accompanied by severe growth reduction, which in many cases can be linked to high SA levels. Indeed, levels of SA were more than 200 times higher in the *dmr6-3_dlo1* double mutant than in the Col-0 control, and ∼20 times higher than in the *dmr6-3* mutant. The single mutant *dmr6-3* showed a modest ∼10 fold increase in SA compared to the Col-0 control, whereas the *dlo1* mutant did not accumulate more SA than Col-0.

To test if the high SA level in *dmr6-3_dlo1* is the cause of the dwarf phenotype and high level of resistance to downy mildew, the double mutant was crossed to the *sid2* mutant, which is strongly compromised in SA biosynthesis as a result of loss of isochorismate synthase 1. The triple mutant *dmr6-3_dlo1_sid2* showed almost complete recovery of the growth phenotype of the *dmr6-3_dlo1* double mutant, although it remained slightly smaller than the *sid2* mutant. Disease assays showed that also the high level of resistance of the *dmr6-3_dlo1* double mutant and *dmr6-3* single mutant was strongly reduced in the absence of SID2. Because of the low SA level, the *sid2* mutant is more susceptible to *H. arabidopsidis* than the wild type Col-0. The level of susceptibility to *H. arabidopsidis* correlates well to the level of total SA in the mutants. Both *dmr6* as well as the *dmr6-3_dlo1* double mutant show no sporulation at 5 dpi and have the highest SA levels. The triple mutant *dmr6-3*_*dlo1_sid2* still produces more SA than Col-0, which might explain it lower susceptibility to downy mildew.

It was concluded that both the resistance to *H. arabidopsidis,* as well as the growth reduction of the *dmr6-3_dlo1* mutant is the result of increased SA levels. The extreme phenotypes of the double mutant demonstrate that the *DLO1* and *DMR6* genes act redundantly. However, the *dmr6* single mutant is more resistant to downy mildew than the *dlo1* mutant. Together with the observed different localization of expression of the *DMR6* and *DLO1* genes, the present data indicate that the *DMR6* and *DLO1* genes have distinct but partially redundant functions as negative regulators of plant immunity.

## Claims

1. Plant belonging to the *Solanaceae* family wherein said plant comprises a genetic trait providing *Phytophthora* resistance and wherein said a genetic trait providing *Phytophthora* resistance is encoded by a combination of at least two genes having a reduced expression, or transcription, of said two genes as compared to said plant belonging to *Solanaceae* family being susceptible to *Phytophthora* wherein:
said plant is sweet pepper (*Capsicum annuum*) or *Capsicum spp.,* said *Phytophthora* resistance is resistance to *Phytophthora capsici* and said combination of at least two genes are genes encoding proteins according to SEQ ID No. 16 and SEQ ID No. 17, or proteins having at least 90% sequence identity with SEQ ID Nos. 16 and 17.

## Patentansprüche

1. Zur der Familie *Solanaceae* gehörende Pflanze, wobei die Pflanze ein genetisches Merkmal aufweist, das *Phytophthora*-Resistenz verleiht, und wobei das genetische Merkmal, das *Phytophthora*-Resistenz verleiht, im Vergleich zu dieser Pflanze, die zu der Familie *Solanaceae* gehört und für *Phytophthora* anfällig ist, durch eine Kombination von wenigstens zwei Genen mit einer reduzierten Expression oder Transkription der zwei Gene kodiert ist, wobei:
diese Pflanze ein Gemüsepaprika (*Capsicium annuum*) oder *Capsicum spp.* ist, wobei die *Phytophthora*-Resistenz eine Resistenz gegen *Phytophthora capsici* ist und die Kombination der wenigstens zwei Gene Gene sind, die Proteine gemäß der SEQ ID No. 16 und SEQ ID No. 17 kodieren, oder Proteine, die wenigstens 90% Sequenzidentität mit den SEQ ID Nos. 16 und 17 haben.

## Revendications

1. Plante appartenant à la famille des *Solanaceae* dans laquelle ladite plante comprend un caractère génétique fournissant une résistance à *Phytophthora* et dans laquelle ledit caractère génétique fournissant une résistance à *Phytophthora* est codé par une combinaison d'au moins deux gènes ayant une expression, ou une transcription, réduite desdits deux gènes par rapport à ladite plante appartenant à la famille des *Solanaceae* étant sensible à *Phytophthora* dans laquelle :
ladite plante est le poivron (*Capsicum annuum*) ou *Capsicum spp.,* ladite résistance à *Phytophthora* est la résistance à *Phytophthora capsici* et ladite combinaison d'au moins deux gènes est des gènes codant pour des protéines selon SEQ ID NO : 16 et SEQ ID NO : 17, ou des protéines ayant une identité de séquence d'au moins 90 % avec SEQ ID NO : 16 et 17.


### SEQUENCE LISTING

<110> Enza Zaden Beheer B.V.
<120> PHYTOPHTHORA RESISTANT PLANTS BELONGING TO THE SOLANACEAE FAMILY
<130> 4/2RV34/52P
<150> PCT/EP2014/062802
   <151> 2014-06-18
<160> 23
<170> BiSSAP 1.2
<210> 1
   <211> 337
   <212> PRT
   <213> Solanum tuberosum
<400> 1 325 330 335
   Asn
<210> 2
   <211> 342
   <212> PRT
   <213> Solanum tuberosum
<400> 2
   Met Glu Thr Thr Ser Val Leu Ser Gly Gly Phe Asn His Ser Thr Leu
   1               5                   10                  15
   Pro Glu Ser Tyr Val Arg Pro Glu Ser Gln Arg Pro Arg Met Ser Glu
               20                  25                  30
   Val Val Asp Arg Asp Asp Leu Val Pro Val Ile Asp Met Ser Cys Thr
           35                  40                  45
   Asp Arg Asn Val Ile Val His Gln Ile Gly Glu Ala Cys Arg Leu Tyr
       50                  55                  60
   Gly Phe Phe Gln Val Ile Asn His Gly Val Ser Lys Lys Val Met Asp
   65                  70                  75                  80
   Glu Met Leu Gly Val Ala His Glu Phe Phe Lys Leu Pro Val Glu Glu
                   85                  90                  95
   Lys Met Lys Leu Tyr Ser Asp Asp Pro Ser Lys Thr Met Arg Leu Ser
               100                 105                 110
   Thr Ser Phe Asn Val Lys Lys Glu Thr Val His Asn Trp Arg Asp Tyr
           115                 120                 125
   Leu Arg Leu His Cys Tyr Pro Leu Asp Lys Tyr Ala Pro Glu Trp Pro
       130                 135                 140
   Ser Asn Pro Pro Ser Phe Arg Glu Ile Val Ser Lys Tyr Cys Met Glu
   145                 150                 155                 160
   Val Arg Gln Val Gly Tyr Arg Leu Glu Glu Ala Ile Ser Glu Ser Leu
                   165                 170                 175
   Gly Leu Glu Lys Asp Cys Ile Lys Asn Val Leu Gly Glu Gln Gly Gln
               180                 185                 190
   His Met Ala Ile Asn Phe Tyr Pro Pro Cys Pro Gln Pro Glu Leu Thr
           195                 200                 205
   Tyr Gly Leu Pro Ala His Thr Asp Pro Asn Ala Ile Thr Ile Leu Leu
       210                 215                 220
   Gln Asp Leu Gln Val Ala Gly Leu Gln Val Leu Lys Asp Gly Glu Trp
   225                 230                 235                 240
   Leu Ser Ile Lys Pro Gln Pro Asp Ala Phe Val Ile Asn Leu Gly Asp
                   245                 250                 255
   Gln Leu Glu Ala Leu Ser Asn Gly Lys Tyr Lys Ser Ile Trp His Arg
               260                 265                 270
   Ala Ile Val Asn Ser Asp Lys Ala Arg Met Ser Val Ala Ser Phe Leu
           275                 280                 285
   Cys Pro Asn Asp Cys Ser Ile Ile Ser Ala Pro Lys Thr Leu Ile Glu
       290                 295                 300
   Asp Gly Ser Ser Ala Ile Tyr Arg Asp Phe Thr Tyr Thr Glu Tyr Tyr
   305                 310                 315                 320
   Asp Lys Phe Trp Ser Arg Asn Leu Asp Gln Glu Tyr Cys Leu Glu Leu
                   325                 330                 335
   Phe Lys Asn Asp Gly Thr
               340
<210> 3
   <211> 340
   <212> PRT
   <213> Petunia
<400> 3
   Met Glu Ser Asn Val Ile Ser Ser Gly Thr Lys Tyr Thr Asn Leu Pro
   1               5                   10                  15
   Lys Ser Tyr Val Arg Pro Glu Ser Gln Arg Pro Arg Leu Ser Glu Val
               20                  25                  30
   Asp Asp Cys Gln Asp Asn Ile Pro Val Ile Asp Leu Cys Cys Arg Asp
           35                  40                  45
   Asn Asn Val Ile Ile Gln Gln Ile Glu Glu Ala Cys Arg Leu Tyr Gly
       50                  55                  60
   Phe Phe Gln Val Ile Asn His Gly Val Pro Lys Lys Leu Ile Glu Glu
   65                  70                  75                  80
   Met Leu Gly Val Ala His Glu Phe Phe Lys Leu Pro Val Glu Glu Lys
                   85                  90                  95
   Met Lys Leu Tyr Ser Asp Asp Pro Ser Lys Thr Met Arg Leu Ser Thr
               100                 105                 110
   Ser Phe Asn Val Lys Lys Glu Thr Val His Asn Trp Arg Asp Tyr Leu
           115                 120                 125
   Arg Leu His Cys Tyr Pro Leu Glu Lys Tyr Ala Pro Glu Trp Pro Ser
       130                 135                 140
   Thr Pro Ser Ser Phe Arg Glu Ile Val Ser Arg Tyr Cys Ile Glu Val
   145                 150                 155                 160
   Arg Gln Leu Gly Tyr Arg Leu Gln Glu Ala Ile Ser Glu Ser Leu Gly
                   165                 170                 175
   Leu Glu Lys Asp Cys Ile Lys Asn Ile Leu Gly Glu Gln Gly Gln His
               180                 185                 190
   Met Ala Val Asn Tyr Tyr Pro Pro Cys Pro Glu Pro Glu Leu Thr Tyr
           195                 200                 205
   Gly Leu Pro Ala His Thr Asp Pro Asn Ala Leu Thr Ile Leu Leu Gln
       210                 215                 220
   Asp Leu Gln Val Ala Gly Leu Gln Val Leu Lys Asp Gly Lys Trp Leu
   225                 230                 235                 240
   Ser Val Lys Pro Arg Ala Asn Ala Phe Val Ile Asn Leu Gly Asp Gln
                   245                 250                 255
   Leu Gln Ala Leu Ser Asn Gly Lys Tyr Arg Ser Val Trp His Arg Ala
               260                 265                 270
   Ile Val Asn Ser Asp Lys Pro Arg Leu Ser Val Ala Ser Phe Leu Cys
           275                 280                 285
   Pro Ser Asp Cys Ala Ile Ile Ser Ala Pro Lys Thr Leu Thr Glu Asp
       290                 295                 300
   Gly Ser Pro Thr Ile Tyr Arg Asp Phe Thr Tyr Pro Glu Tyr Tyr Lys
   305                 310                 315                 320
   Lys Phe Trp Ser Arg Asn Leu Asp Gln Glu His Cys Met Glu Leu Phe
                   325                 330                 335
   Lys Lys Gly Ser
               340
<210> 4
   <211> 337
   <212> PRT
   <213> Petunia
<400> 4
   Met Glu Thr Lys Val Leu Ser Ser Gly Ile Arg His Ser Thr Leu Pro
   1               5                   10                  15
   Gln Asn Tyr Val Arg Pro Lys Ser Asp Arg Pro Arg Leu Ser Glu Val
               20                  25                  30
   Ala Asn Cys Glu Asn Val Pro Val Ile Asp Leu Gly Cys Ala Asp Arg
           35                  40                  45
   Thr Leu Ile Ile His Gln Ile Ser Glu Ala Cys Arg Leu Tyr Gly Phe
       50                  55                  60
   Phe Gln Val Ile Asn His Gly Val Pro Lys Lys Ile Val Glu Glu Met
   65                  70                  75                  80
   Leu Glu Ile Ala Gly Glu Phe Phe Arg Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Thr Val His Asn Trp Arg Asp Tyr Leu Arg
           115                 120                 125
   Leu His Cys Tyr Pro Leu Glu Lys Tyr Ala Pro Glu Trp Pro Ser Asn
       130                 135                 140
   Pro Ser Ser Phe Arg Glu Ile Val Ser Arg Tyr Cys Thr Glu Val Arg
   145                 150                 155                 160
   Gln Leu Gly Phe Arg Leu Gln Glu Ala Ile Ala Glu Ser Leu Gly Leu
                   165                 170                 175
   Glu Lys Glu Cys Ile Lys Asp Val Leu Gly Glu Gln Gly Gln His Met
               180                 185                 190
   Ala Ile Asn Phe Tyr Pro Pro Cys Pro Glu Pro Glu Leu Thr Tyr Gly
           195                 200                 205
   Leu Pro Ala His Thr Asp Pro Asn Ala Leu Thr Ile Leu Leu Gln Asp
       210                 215                 220
   Leu Gln Val Ala Gly Leu Gln Val Leu Lys Asp Gly Lys Trp Leu Ala
   225                 230                 235                 240
   Val Lys Pro Gln Pro Asp Ala Phe Val Val Asn Leu Gly Asp Gln Leu
                   245                 250                 255
   Gln Ala Val Ser Asn Gly Arg Tyr Lys Ser Val Trp His Arg Ala Val
               260                 265                 270
   Val Asn Thr Glu Asn Ala Arg Met Ser Val Ala Ser Phe Leu Cys Pro
           275                 280                 285
   Cys Asp Ser Ala Lys Ile Ser Ala Pro Lys Leu Leu Thr Asp Asp Gly
       290                 295                 300
   Ser Pro Ile Ile Tyr Arg Asp Phe Thr Tyr Ala Glu Tyr Tyr Lys Lys
   305                 310                 315                 320
   Phe Trp Ser Arg Asn Leu Asp Gln Glu His Cys Leu Glu Leu Phe Lys
                   325                 330                 335
   Asn
<210> 5
   <211> 337
   <212> PRT
   <213> Solanum lycopersicum
<400> 5
   Met Glu Thr Lys Val Ile Ser Ser Gly Ile Asn His Ser Thr Leu Pro
   1               5                   10                  15
   Gln Ser Tyr Ile Arg Pro Glu Ser Asp Arg Pro Arg Leu Ser Glu Val
               20                  25                  30
   Val Asp Cys Glu Asn Val Pro Ile Ile Asp Leu Ser Cys Gly Asp Gln
           35                  40                  45
   Ala Gln Ile Ile Arg Gln Ile Gly Glu Ala Cys Gln Thr Tyr Gly Phe
       50                  55                  60
   Phe Gln Val Ile Asn His Gly Val Pro Lys Glu Val Val Glu Lys Met
   65                  70                  75                  80
   Leu Gly Val Ala Gly Glu Phe Phe Asn Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Thr Val His Asn Trp Arg Asp Tyr Leu Arg
           115                 120                 125
   Leu His Cys Tyr Pro Leu Glu Lys Tyr Ala Pro Glu Trp Pro Ser Asn
       130                 135                 140
   Pro Ser Ser Phe Arg Glu Ile Val Ser Arg Tyr Cys Arg Glu Ile Arg
   145                 150                 155                 160
   Gln Leu Gly Phe Arg Leu Glu Glu Ala Ile Ala Glu Ser Leu Gly Leu
                   165                 170                 175
   Asp Lys Glu Cys Ile Lys Asp Val Leu Gly Glu Gln Gly Gln His Met
               180                 185                 190
   Ala Ile Asn Tyr Tyr Pro Pro Cys Pro Gln Pro Glu Leu Thr Tyr Gly
           195                 200                 205
   Leu Pro Ala His Thr Asp Pro Asn Ser Leu Thr Ile Leu Leu Gln Asp
       210                 215                 220
   Leu Gln Val Ala Gly Leu Gln Val Leu Lys Asp Gly Lys Trp Leu Ala
   225                 230                 235                 240
   Val Lys Pro Gln Pro Asp Ala Phe Val Ile Asn Leu Gly Asp Gln Leu
                   245                 250                 255
   Gln Ala Val Ser Asn Gly Lys Tyr Arg Ser Val Trp His Arg Ala Ile
               260                 265                 270
   Val Asn Ser Asp Gln Ala Arg Met Ser Val Ala Ser Phe Leu Cys Pro
           275                 280                 285
   Cys Asp Ser Ala Lys Ile Ser Ala Pro Lys Leu Leu Thr Glu Asp Gly
       290                 295                 300
   Ser Pro Val Ile Tyr Gln Asp Phe Thr Tyr Ala Glu Tyr Tyr Asn Lys
   305                 310                 315                 320
   Phe Trp Ser Arg Asn Leu Asp Gln Gln His Cys Leu Glu Leu Phe Lys
                   325                 330                 335
   Asn
<210> 6
   <211> 320
   <212> PRT
   <213> Solanum lycopersicum
<400> 6
   Met Met Thr Thr Thr Ser Val Leu Ser Ser Gly Phe Asn His Ser Thr
   1 5 10 15
   Leu Pro Gln Ser Tyr Val Arg Pro Glu Ser Gln Arg Pro Cys Met Ser
   20 25 30
   Glu Val Val Asp Ser Asp Asp Leu Val Pro Val Ile Asp Met Ser Cys
   35 40 45
   Thr Asp Arg Asn Val Ile Val His Gln Ile Gly Glu Ala Cys Arg Leu
   50 55 60
   Tyr Gly Phe Phe Gln Val Ile Asn His Gly Val Ser Lys Lys Ala Met
   65 70 75 80
   Asp Glu Met Leu Gly Thr Met Arg Leu Ser Thr Ser Phe Asn Val Lys
   85 90 95
   Lys Glu Thr Val His Asn Trp Arg Asp Tyr Leu Arg Leu His Cys Tyr
   100 105 110
   Pro Leu Asp Lys Tyr Ala Pro Glu Trp Pro Ser Asn Pro Pro Ser Phe
   115 120 125
   Arg Glu Ile Val Ser Lys Tyr Cys Met Glu Val Arg Glu Leu Gly Tyr
   130 135 140
   Arg Leu Glu Glu Ala Ile Ser Glu Ser Leu Gly Leu Glu Lys Asp Cys
   145 150 155 160
   Ile Lys Asn Val Leu Gly Glu Gln Gly Gln His Met Ala Ile Asn Phe
   165 170 175
   Tyr Pro Gln Cys Pro Gln Pro Glu Leu Thr Tyr Gly Leu Pro Ala His
   180 185 190
   Thr Asp Pro Asn Ala Ile Thr Ile Leu Leu Gln Asp Leu Gln Val Ala
   195 200 205
   Gly Leu Gln Val Leu Lys Asp Gly Lys Trp Leu Ser Ile Lys Pro Gln
   210 215 220
   Pro Asn Ala Phe Val Ile Asn Leu Gly Asp Gln Leu Glu Ala Leu Ser
   225 230 235 240
   Asn Gly Lys Tyr Lys Ser Ile Trp His Arg Ala Ile Val Asn Ser Asp
   245 250 255
   Lys Ala Arg Met Ser Val Ala Ser Phe Leu Cys Pro Asn Asp Cys Ser
   260 265 270
   Ile Ile Ser Ala Pro Lys Thr Leu Thr Glu Asp Gly Ser Ser Ala Ile
   275 280 285
   Tyr Arg Asp Phe Thr Tyr Ala Glu Tyr Tyr Glu Lys Phe Trp Ser Arg
   290 295 300
   Asn Leu Asp Gln Glu Tyr Cys Leu Glu Leu Phe Lys Asn Asp Gly Thr
   305 310 315 320
<210> 7
   <211> 1014
   <212> DNA
   <213> Solanum tuberosum
<220>
   <221> source
   <222> 1..1014
   <223> /mol_type="unassigned DNA" /organism="Solanum tuberosum"
<400> 7
   atggaaacga aagttatttc cagcggaatc caccactcta ctctccctca aagttacatc      60
   cgacccgaat ccgataggcc acgtctatcg gatgtggtcg attgcgaaaa tgttccaata     120
   attgacttag gttgcggaga ccaagctcaa ataatccgtc taattggaga agcttgtcaa     180
   acttatggtt tctttcaggt aattaatcat ggtgtaccaa aggaagttgt agagaaaatg     240
   ctaggggtag ctggggaatt tttcaatcta ccagtagaag agaagctaaa attgtattca     300
   gatgatcctt caaagaccat gagattatct actagtttta atgttaaaaa ggagacagtt     360
   cataattgga gagattatct cagacttcat tgtcatcctc tggagaaata tgctcctgaa     420
   tggccttcta atccatcgtc tttcagggat atcgtgagca gatattgcac ggaagttcga     480
   caactcggat ttagattgga ggaagccata gcagagagcc tgggcttaga gaaagagtgt     540
   attaaagatg tattgggaga acaaggccaa catatggcta tcaattttta tcctccttgt     600
   ccacaaccag aactcacata tgggcttccg gcccatactg atccaaattc acttacaatt     660
   cttcttcaag acttgcaagt ttctggtctt caagttctta aagatggtaa atggttggct     720
   gtcaaacctc aaccagatgc ctttgtcatt aatcttggtg atcaattgca ggcagtaagt     780
   aacggtaagt acaaaagtgt atggcatcga gctattgtga attcagatca agctaggatg     840
   tcagtggctt cgttcctatg tccgtgcgat agcgcgaaaa tcagtgctcc aaaactcctg     900
   acagaagatg gatctccagt catttatcag gacttcacgt atgctgagta ttacaagaag     960
   ttctggagca ggaatttgga ccaggaacat tgtttggaac ttttcaagaa ttaa          1014
<210> 8
   <211> 1029
   <212> DNA
   <213> Solanum tuberosum
<220>
   <221> source
   <222> 1..1029
   <223> /mol_type="unassigned DNA" /organism="Solanum tuberosum"
<400> 8
   atggaaacaa caagtgttct ttccggtgga ttcaaccact caaccctccc tgaatcttac      60
   gttcgacctg aatcccaaag accccgcatg tctgaagttg ttgatcgtga tgatcttgtt     120
   ccagttatcg atatgtcttg tactgatagg aacgttatcg ttcatcaaat tggcgaagct     180
   tgtcgccttt atgggttttt ccaggtgata aatcacggtg tatcaaagaa ggttatggat     240
   gaaatgttgg gggtagctca tgaatttttt aagcttccag tggaagaaaa gatgaaattg     300
   tactcagatg atccatcaaa gactatgaga ttatcaacta gttttaatgt taagaaggaa     360
   actgttcata attggagaga ttatcttagg ctacactgtt atcctttgga caaatatgcc     420
   cctgaatggc cttctaatcc tccttctttc agggaaatag tgagcaaata ttgcatggaa     480
   gttagacaag ttggatatag attagaagaa gcaatatcag agagcctagg gctcgagaaa     540
   gattgtatta aaaatgtgtt gggtgaacaa ggacaacata tggctatcaa tttttatcct     600
   ccatgtccac aacctgaact aacttatggg ttaccagccc atacagatcc aaatgcaatt     660
   acaattcttc ttcaagattt gcaagtggct ggccttcaag ttcttaagga tggagaatgg     720
   ttatctatta aacctcaacc tgatgccttt gtcatcaatc ttggtgatca attggaggca     780
   ttgagtaatg gaaagtataa aagtatatgg catagagcta ttgtaaattc agataaagca     840
   aggatgtctg tggcttcttt cctctgtccc aatgattgtt ccattatcag tgctccaaaa     900
   accttaattg aagatggatc ttcagccatt tatcgagatt tcacttatac tgaatattat     960
   gacaaatttt ggagcaggaa tttagaccag gaatattgtt tagaactttt caagaacgat    1020
   ggaacctag                                                            1029
<210> 9
   <211> 1014
   <212> DNA
   <213> Petunia
<220>
   <221> source
   <222> 1..1014
   <223> /mol_type="unassigned DNA" /organism="Petunia"
<400> 9
   atggaaacaa aagttctttc aagtggaatc cgtcattcta ccctccctca aaattatgtc      60
   cgacccaaat ccgataggcc acgtctttca gaagtggcca attgtgaaaa cgttccagtt     120
   attgacttgg gttgtgctga cagaactctc ataattcatc aaattagcga agcctgtcgt     180
   ctttatggtt ttttccaggt aataaaccat ggtgtaccaa aaaaaatagt tgaggaaatg     240
   ctagagatag ctggggagtt ttttaggcta ccagttgaag agaagcttaa gttgtattca      300
   gatgaccctt caaagaccat gagattatca actagtttta atgtaaagaa ggagacggtg      360
   cacaattgga gagattatct cagacttcat tgttatcctc tggagaaata tgctcctgaa      420
   tggccttcaa atccttcatc tttcagggaa atcgtgagca gatattgcac ggaagttcga      480
   caacttggat tcagattgca agaagccata gcagaaagct taggcttaga gaaagagtgt      540
   ataaaggatg tgttaggtga acaaggtcaa catatggcta taaactttta tcctccatgc      600
   ccagaaccag aactcactta cgggctgcca gcccataccg atccaaatgc tcttacaatt      660
   cttcttcaag acttgcaagt agctggtctc caagttctta aagatggcaa atggttggct      720
   gtcaaacctc agcccgatgc ctttgttgtt aatctcggtg atcaactgca ggcagtgagt      780
   aacggaaggt acaaaagcgt atggcatcga gctgttgtaa atacagaaaa tgccaggatg      840
   tctgtggctt cgttcttatg tccctgtgat agtgcaaaaa tcagtgctcc aaaactcctc      900
   actgatgatg gatctccaat aatttatcgg gacttcacgt atgcagagta ttacaagaag      960
   ttctggagca ggaatttgga ccaagaacat tgtttggaac ttttcaagaa ttaa           1014
<210> 10
   <211> 1023
   <212> DNA
   <213> Petunia
<220>
   <221> source
   <222> 1..1023
   <223> /mol_type="unassigned DNA" /organism="Petunia"
<400> 10
   atggaatcta atgttatttc cagcggaacc aaatacacaa acctccctaa aagttatgtt      60
   cgcccagaat cccaacgacc tcggttatct gaagtagacg attgccaaga taatattcca     120
   gttattgatt tgtgttgcag agacaataac gttatcattc aacaaattga agaagcttgt     180
   cgtctttatg gcttttttca ggtaataaac catggtgtac caaagaaact aatagaggaa    240
   atgctagggg tagctcatga gtttttcaag ctaccagtgg aagagaagat gaagttgtac     300
   tcagatgatc catcaaagac catgagatta tcaacaagtt ttaatgtgaa gaaggaaact     360
   gttcataatt ggagagacta tcttagattg cactgctatc ctttggagaa atatgcccct     420
   gaatggcctt ctactccctc ttctttcagg gaaatcgtta gcagatattg catagaagtt     480
   cgacaacttg gatatagatt acaagaagca atatcagaga gcttaggcct agagaaagat     540
   tgtataaaaa atatattggg tgaacaaggt caacatatgg ctgttaatta ttaccctcca     600
   tgtccagaac cagaactaac ttatggtttg ccagcccata ctgatcctaa tgcccttact     660
   atacttcttc aagacttgca agtagcaggt cttcaagttc tcaaggatgg taaatggtta     720
   tctgtgaaac ctcgggccaa tgcctttgtc atcaatcttg gtgatcaatt gcaggcgctg     780
   agtaatggaa aatatagaag tgtatggcac agagctatag taaattcaga caaaccaagg     840
   ctgtcagtgg cttctttctt gtgtcctagt gattgtgcga taatcagtgc tccaaaaacc     900
   ttaactgaag atgggtctcc aaccatttat cgggatttca cgtatccaga atattacaag     960
   aaattttgga gcagaaattt agatcaagaa cactgtatgg aacttttcaa gaaaggaagc    1020
   tag                                                                   1023
<210> 11
   <211> 1014
   <212> DNA
   <213> Solanum lycopersicum
<220>
   <221> source
   <222> 1..1014
   <223> /mol_type="unassigned DNA" /organism="Solanum lycopersicum"
<400> 11
   atggaaacca aagttatttc tagcggaatc aaccactcta ctcttcctca aagttacatc      60
   cgacccgaat ccgatagacc acgtctatcg gaagtggtcg attgtgaaaa tgttccaata     120
   attgacttaa gttgcggaga tcaagctcaa ataattcgtc aaattggaga agcttgtcaa     180
   acttatggtt tctttcaggt aattaatcat ggtgtaccaa aggaagttgt agagaaaatg     240
   ctaggggtag ctggggaatt tttcaattta ccagtagaag agaaactaaa attatattca     300
   gatgatcctt caaagaccat gagattatca acaagtttta atgttaaaaa ggagacagtt     360
   cataattgga gagattatct cagacttcat tgttatcctc tagagaagta tgctcctgaa     420
   tggccttcta atccatcatc tttcagggaa atcgtgagca gatattgcag ggaaattcgt     480
   caactcggat ttagattaga agaagccata gcagaaagcc tggggttaga taaagagtgt     540
   ataaaagatg tattgggtga acaaggacaa catatggcta tcaattatta tcctccttgt     600
   ccacaaccag aacttactta tgggcttccg gcccatactg atccaaattc acttacaatt     660
   cttcttcaag acttgcaagt tgcgggtctt caagttctta aagatggcaa atggttagct     720
   gtaaaacctc aacctgacgc ctttgtcatt aatcttgggg atcaattgca ggcagtaagt     780
   aacggtaagt acagaagtgt atggcatcga gctattgtga attcagatca agctaggatg     840
   tcagtggctt cgtttctatg tccgtgtgat agcgcgaaaa tcagtgcacc aaagctgctg     900
   acagaagatg gatctccagt gatttatcaa gactttacgt atgctgagta ttacaacaag     960
   ttctggagca ggaatttgga ccagcaacat tgtttggaac ttttcaagaa ctaa          1014
<210> 12
   <211> 963
   <212> DNA
   <213> Solanum lycopersicum
<220>
   <221> source
   <222> 1..963
   <223> /mol_type="unassigned DNA" /organism="Solanum lycopersicum"
<400> 12
   atgatgacaa caacaagtgt tctttctagt ggattcaacc actcaaccct ccctcaatct      60
   tacgttcgac ctgaatctca aagaccttgc atgtctgaag ttgttgatag cgacgatctt     120
   gtcccagtca ttgatatgtc ttgtactgat aggaacgtta tcgttcatca aatcggtgaa     180
   gcttgtcgtc tttatgggtt ttttcaggtg ataaatcacg gtgtgtcgaa gaaggcgatg     240
   gatgaaatgt tagggactat gagattatca actagtttta atgttaagaa ggaaactgtt     300
   cataattgga gagattatct taggctacat tgttatcctt tggacaaata tgcccctgaa     360
   tggccttcta atcctccttc tttcagggaa atagtaagca aatattgcat ggaagttaga     420
   gagcttggat atagattgga agaagcaata tcagagagct tagggcttga gaaggattgt     480
   ataaaaaatg tgttaggtga acaaggacaa catatggcta tcaattttta tcctcagtgt     540
   ccacaacctg aattaactta tgggttacca gcccatacag atccaaatgc aattacaatt     600
   cttcttcaag atttgcaagt ggctggcctt caagttctta aggatggaaa atggttatct     660
   attaaacctc agcctaatgc ctttgtcatc aatcttggtg atcaattgga ggcgttgagt     720
   aatgggaagt ataaaagtat atggcataga gctattgtaa attcagacaa agcaaggatg     780
   tctgtggctt cttttctctg tcccaatgat tgttccatta tcagtgctcc aaaaacctta     840
   actgaagatg gatcttctgc aatttatcga gatttcactt atgctgaata ttatgaaaaa     900
   ttctggagca ggaatttaga tcaggaatat tgtttagaac tttttaagaa cgatggaacc     960
   tag                                                                   963
<210> 13
   <211> 34
   <212> DNA
   <213> Artificial Sequence
<220>
   <221> source
   <222> 1..34
   <223> /mol_type="unassigned DNA" /note="S. Lycopersicum AttB1-F primer" /organism="Artificial Sequence"
<400> 13
   aaaaagcagg cttcttgggt gaacaaggac aaca 34
<210> 14
   <211> 33
   <212> DNA
   <213> Artificial Sequence
<220>
   <221> source
   <222> 1..33
   <223> /mol_type="unassigned DNA" /note="S.Lycopersicum AttB2-R primer" /organism="Artificial Sequence"
<400> 14
   agaaagctgg gtaaaacgaa gccactgaca tcc 33
<210> 15
   <211> 303
   <212> DNA
   <213> Artificial Sequence
<220>
   <221> source
   <222> 1..303
   <223> /mol_type="unassigned DNA" /note="silencing construct for SEQ ID Nos 11 and 12" /organism="Artificial Sequence"
<400> 15
   ttgggtgaac aaggacaaca tatggctatc aattattatc ctccttgtcc acaaccagaa     60
   cttacttatg ggcttccggc ccatactgat ccaaattcac ttacaattct tcttcaagac    120
   ttgcaagttg cgggtcttca agttcttaaa gatggcaaat ggttagctgt aaaacctcaa    180
   cctgacgcct ttgtcattaa tcttggggat caattgcagg cagtaagtaa cggtaagtac    240
   agaagtgtat ggcatcgagc tattgtgaat tcagatcaag ctaggatgtc agtggcttcg    300
   ttt                                                                  303
<210> 16
   <211> 375
   <212> PRT
   <213> Capsicum annuum
<400> 16
   Tyr Phe Ser Ile Asp Leu Cys Ala Ala Ser Tyr Ile Ala Thr Asp Ser
   1               5                   10                  15
   Met Glu Thr Lys Val Ile Ser Ser Gly Ile Arg His Ser Thr Leu Pro
               20                  25                  30
   Gln Ser Tyr Ile Arg Pro Glu Ser Asp Arg Pro Arg Leu Ser Glu Val
           35                  40                  45
   Ala Asp Cys Glu Asn Val Pro Val Ile Asp Leu Gly Cys Thr Asp Arg
       50                  55                  60
   Thr His Ile Ile Arg Gln Ile Gly Glu Ala Cys Arg Asn Tyr Gly Phe
   65                  70                  75                  80
   Phe Gln Val Ile Asn His Gly Val Pro Lys Glu Ile Val Glu Gln Met
                   85                  90                  95
   Leu Glu Val Ala Gly Glu Phe Phe Arg Leu Pro Val Glu Glu Lys Leu
               100                 105                 110
   Lys Leu Tyr Ser Asp Asp Pro Ser Lys Thr Met Arg Leu Ser Thr Ser
           115                 120                 125
   Phe Asn Val Lys Lys Glu Thr Val His Asn Trp Arg Asp Tyr Leu Arg
       130                 135                 140
   Leu His Cys Tyr Pro Leu Glu Lys Tyr Ala Pro Glu Trp Pro Ser Asn
   145                 150                 155                 160
   Pro Ser Ser Phe Ser Tyr Arg Glu Ile Val Ser Arg Tyr Cys Thr Glu
                   165                 170                 175
   Val Arg Gln Leu Gly Phe Arg Leu Gln Glu Ala Ile Ala Glu Ser Leu
               180                 185                 190
   Gly Leu Glu Lys Glu Cys Ile Lys Asp Val Leu Gly Glu Gln Gly Gln
           195                 200                 205
   His Met Ala Ile Asn Phe Tyr Pro Pro Cys Pro Gln Pro Glu Leu Thr
       210                 215                 220
   Tyr Gly Leu Pro Ala His Thr Asp Pro Asn Ser Leu Thr Ile Leu Leu
   225                 230                 235                 240
   Gln Asp Leu Gln Val Ala Gly Leu Gln Val Leu Lys Asp Gly Lys Trp
                   245                 250                 255
   Leu Ala Val Lys Pro Gln Pro Asp Ala Phe Val Ile Asn Leu Gly Asp
               260                 265                 270
   Gln Leu Gln Ala Val Ser Asn Gly Lys Tyr Lys Ser Val Trp His Arg
           275                 280                 285
   Ala Ile Val Asn Ser Asp Lys Ala Arg Met Ser Val Ala Ser Phe Leu
       290                 295                 300
   Cys Pro Cys Asp Ser Ala Lys Ile Ser Ala Pro Lys Leu Leu Thr Glu
   305                 310                 315                 320
   Asp Gly Ser Pro Val Ile Tyr Arg Asp Phe Thr Tyr Ala Glu Tyr Tyr
                   325                 330                 335
   Lys Lys Phe Trp Ser Arg Asn Leu Asp Gln Glu His Cys Leu Glu Leu
               340                 345                 350
   Phe Lys Asn Lys Gly Arg Leu Ala Asp Gln Ile Arg Trp Ile Ser Asp
           355                 360                 365
   Val Ser Asn Ile Tyr Thr Leu
       370                 375
<210> 17
   <211> 342
   <212> PRT
   <213> Capsicum annuum
<400> 17
   Met Glu Thr Lys Asn Val Leu Ser Ser Gly Thr Lys Tyr Ser Thr Leu
   1               5                   10                  15
   Pro Glu Thr Tyr Ile Arg Pro Glu Ser Gln Arg Pro Arg Leu Ser Glu
               20                  25                  30
   Val Val Asp Cys Glu Asp Phe Ile Pro Val Ile Asp Met Ser Cys Thr
           35                  40                  45
   Asp Arg Asn Ile Ile Val His Gln Ile Gly Gln Ala Cys Leu Leu Tyr
       50                  55                  60
   Gly Phe Phe Gln Val Ile Asn His Asp Val Pro Lys Glu Val Ile Glu
   65                  70                  75                  80
   Gly Met Leu Gly Val Ala His Glu Phe Phe Lys Leu Pro Val Glu Glu
                   85                  90                  95
   Lys Met Lys Leu Tyr Ser Asp Asp Pro Ser Lys Thr Met Arg Leu Ser
               100                 105                 110
   Thr Ser Phe Asn Val Lys Lys Glu Thr Val His Asn Trp Arg Asp Tyr
           115                 120                 125
   Leu Arg Leu His Cys Tyr Pro Leu Asp Lys Tyr Ala Pro Glu Trp Pro
       130                 135                 140
   Ser Asn Pro Ser Ser Phe Arg Glu Ile Val Ser Lys Tyr Cys Met Glu
   145                 150                 155                 160
   Val Arg Gln Leu Gly Tyr Arg Leu Glu Glu Ala Ile Ser Glu Ser Leu
                   165                 170                 175
   Gly Leu Gly Lys Asp Cys Ile Lys Asn Val Leu Gly Glu Gln Gly Gln
               180                 185                 190
   His Met Ala Ile Asn Phe Tyr Pro Gln Cys Pro Gln Pro Glu Leu Thr
           195                 200                 205
   Tyr Gly Leu Pro Ala His Thr Asp Pro Asn Ala Ile Thr Ile Leu Leu
       210                 215                 220
   Gln Asp Leu Gln Val Glu Gly Leu Gln Val Leu Lys Asp Gly Lys Trp
   225                 230                 235                 240
   Leu Ser Val Lys Pro Gln Pro Asn Ala Phe Val Ile Asn Leu Gly Asp
                   245                 250                 255
   Gln Leu Gln Ala Leu Ser Asn Gly Lys Tyr Lys Ser Val Trp His Arg
               260                 265                 270
   Ala Ile Val Asn Ser Asp Lys Ala Arg Met Ser Val Ala Ser Phe Leu
           275                 280                 285
   Cys Pro Ser Asp Cys Ser Ile Ile Ser Ala Pro Lys Ala Leu Thr Glu
       290                 295                 300
   Asp Gly Ser Ser Ala Ile Tyr Arg Asp Phe Thr Tyr Thr Glu Tyr Tyr
   305                 310                 315                 320
   Asn Lys Phe Trp Ser Arg Ser Leu Asp Gln Glu Arg Arg Leu Lys Leu
                   325                 330                 335
   Phe Lys Lys Val Tyr Thr
               340
<210> 18
   <211> 337
   <212> PRT
   <213> Nicotiana benthamiana
<400> 18
   Met Glu Ala Lys Val Leu Ser Ser Gly Ile Arg His Ser Thr Ile Pro
   1               5                   10                  15
   Gln Ser Tyr Ile Arg Pro Gln Ser Asp Arg Pro Arg Leu Ser Glu Val
               20                  25                  30
   Ala Asp Cys Glu Asn Val Pro Val Val Asp Ile Gly Cys Gly Asp Arg
           35                  40                  45
   Asn Leu Ile Val His Gln Ile Gly Glu Ala Cys Arg Leu Tyr Gly Phe
       50                  55                  60
   Phe Gln Val Ile Asn His Gly Val Pro Lys Asn Leu Ile Asp Glu Met
   65                  70                  75                  80
   Leu Glu Ile Ala Gly Glu Phe Phe Arg Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Lys Val His Asn Trp Arg Asp Tyr Leu Arg
           115                 120                 125
   Leu His Cys Tyr Pro Leu Glu Asn Tyr Ala Pro Glu Trp Pro Ser Asn
       130                 135                 140
   Pro Ser Ser Phe Arg Glu Ile Val Ser Arg Tyr Cys Met Glu Val Arg
   145                 150                 155                 160
   Gln Leu Gly Phe Arg Leu Gln Glu Ala Ile Ala Glu Ser Leu Gly Leu
                   165                 170                 175
   Glu Lys Glu Cys Ile Lys Asp Val Leu Gly Glu Gln Gly Gln His Met
               180                 185                 190
   Ala Ile Asn Phe Tyr Pro Pro Cys Pro Gln Pro Glu Leu Thr Tyr Gly
           195                 200                 205
   Leu Pro Ala His Thr Asp Pro Asn Ala Leu Thr Ile Leu Leu Gln Asp
       210                 215                 220
   Leu Glu Val Ala Gly Leu Gln Val Leu Lys Asp Gly Glu Trp Leu Ala
   225                 230                 235                 240
   Val Lys Pro Gln Pro Asp Ala Phe Val Ile Asn Leu Gly Asp Gln Leu
                   245                 250                 255
   Gln Ala Val Ser Asn Gly Arg Tyr Lys Ser Val Trp His Arg Ala Ile
               260                 265                 270
   Val Asn Ser Asp Lys Ala Arg Leu Ser Val Ala Ser Phe Leu Cys Pro
           275                 280                 285
   Cys Asp Ser Ala Lys Ile Ser Ala Pro Lys Leu Leu Thr Glu Asp Gly
       290                 295                 300
   Ser Pro Val Ile Tyr Gln Asp Phe Thr Tyr Ala Glu Tyr Tyr Lys Lys
   305                 310                 315                 320
   Phe Trp Ser Arg Asn Leu Asp Gln Glu His Cys Leu Glu Leu Phe Lys
                   325                 330                 335
   Asn
<210> 19
   <211> 337
   <212> PRT
   <213> Nicotiana tabacum
<400> 19
   Met Glu Thr Lys Val Leu Ser Ser Gly Ile Arg His Ser Thr Ile Pro
   1               5                   10                  15
   Gln Ser Tyr Ile Arg Pro Gln Ser Asp Arg Pro Arg Leu Ser Glu Val
               20                  25                  30
   Val Asp Cys Glu Asn Val Pro Val Ile Asp Met Gly Cys Gly Asp Arg
           35                  40                  45
   Asn His Ile Ile Arg Gln Ile Gly Glu Ala Cys His Leu Tyr Gly Phe
       50                  55                  60
   Phe Gln Val Ile Asn His Gly Val Pro Thr Asn Leu Val Glu Glu Met
   65                  70                  75                  80
   Leu Glu Ile Ala Arg Glu Phe Phe Asn Leu Pro Val Glu Glu Lys Leu
                   85                  90                  95
   Lys Leu Tyr Ser Asp Asp Pro Ser Lys Thr Met Arg Leu Ser Thr Ser
               100                 105                 110
   Phe Asn Val Lys Lys Glu Thr Val His Asn Trp Arg Asp Tyr Leu Arg
           115                 120                 125
   Leu His Cys Tyr Pro Leu Glu Lys Tyr Ala Pro Glu Trp Pro Ser Asn
       130                 135                 140
   Pro Ala Ser Phe Arg Glu Ile Val Ser Arg Tyr Cys Thr Glu Val Arg
   145                 150                 155                 160
   Gln Leu Gly Phe Arg Leu Gln Glu Ala Ile Ala Glu Ser Leu Gly Leu
                   165                 170                 175
   Glu Lys Val Cys Ile Lys Asp Val Leu Gly Glu Gln Gly Gln His Met
               180                 185                 190
   Ala Ile Asn Phe Tyr Pro Pro Cys Pro Gln Pro Glu Leu Thr Tyr Gly
           195                 200                 205
   Leu Pro Ala His Thr Asp Pro Asn Ala Leu Thr Ile Leu Leu Gln Asp
       210                 215                 220
   Leu Gln Val Ala Gly Leu Gln Val Leu Lys Asp Gly Lys Trp Leu Ala
   225                 230                 235                 240
   Val Lys Pro Gln Pro Asp Ala Phe Val Ile Asn Leu Gly Asp Gln Leu
                   245                 250                 255
   Gln Ala Val Ser Asn Gly Arg Tyr Lys Ser Val Trp His Arg Ala Ile
               260                 265                 270
   Val Asn Ser Asp Lys Ala Arg Leu Ser Val Ala Ser Phe Leu Cys Pro
           275                 280                 285
   Cys Asp Ser Ala Lys Ile Ser Ala Pro Lys Leu Leu Thr Glu Asp Gly
       290                 295                 300
   Ser Pro Val Ile Tyr Gln Asp Phe Thr Tyr Ala Glu Tyr Tyr Lys Lys
   305                 310                 315                 320
   Phe Trp Ser Arg Asn Leu Asp Gln Glu His Cys Leu Glu Phe Phe Lys
                   325                 330                 335
   Asn
<210> 20
   <211> 1139
   <212> DNA
   <213> Capsicum annuum
<220>
   <221> source
   <222> 1..1139
   <223> /mol_type="unassigned DNA" /organism="Capsicum annuum"
<400> 20
   tatttttcaa ttgatttatg tgcagcatca tatatagcca cagattccat ggaaaccaaa 60
   gttatttcca gcggaatccg ccactctact cttcctcaaa gttatatccg acccgaatcc 120
   gataggccac gcctttctga agtggctgat tgtgaaaatg ttccggttat cgacttaggc 180
   tgcactgacc gaactcacat aattcgtcaa atcggcgaag cttgtcgaaa ttatggcttc 240
   ttccaggtaa ttaatcatgg tgtaccaaag gaaattgtag aacaaatgct agaggtagct 300
   ggggaatttt tcaggttacc agtagaagag aagctgaaat tatattcaga tgacccttca 360
   aagaccatga gattatctac aagttttaat gttaaaaaag agacggttca caattggaga 420
   gattatctca ggcttcactg ttatcctctt gaaaaatatg cccctgaatg gccttctaat 480
   ccctcatctt tcagctacag ggaaatagtg agcagatact gcacggaagt tcgacaactt 540
   ggattcagat tgcaggaagc aatagcagag agcttaggct tagagaaaga gtgtataaag 600
   gatgtattgg gggaacaagg tcaacatatg gctatcaact tttatcctcc atgtccacaa 660
   ccagaactca cttatggact tccagctcat actgatccaa attcacttac tattcttcta 720
   caagacttgc aagtagctgg ccttcaggtt cttaaagatg gcaaatggtt agctgtcaaa 780
   cctcagccag atgcctttgt cattaatctt ggtgatcaat tgcaggcagt gagtaacgga 840
   aagtacaaaa gcgtatggca tagagctatt gtaaattcag acaaagccag gatgtcagtg 900
   gcttcgttcc tttgcccatg tgatagcgcg aaaatcagtg ctccaaaact cctcactgaa 960
   gatggatctc cagtcattta tcgggatttc acctatgctg aatattacaa gaagttctgg 1020
   agcaggaatt tggatcagga acattgtttg gaacttttca agaattaaaa gggccgtttg 1080
   gctgatcaga taaggtggat ctcctaggat taagtttcaa actaaattta tactttgtt 1139
<210> 21
   <211> 1029
   <212> DNA
   <213> Capsicum annuum
<220>
   <221> source
   <222> 1..1029
   <223> /mol_type="unassigned DNA" /organism="Capsicum annuum"
<400> 21
   atggaaacaa aaaatgttct ttctagtgga actaagtact caactctccc tgaaacgtac 60
   attcgccctg aatcccaaag acctcgtctg tctgaagttg ttgattgtga agattttatt      120
   cctgttatcg atatgtcttg cactgacagg aacattatcg ttcatcaaat tggccaagct      180
   tgtcttcttt acgggttttt ccaggtgata aatcatgacg tgccaaagga ggtgatagag      240
   ggaatgctag gggtagctca tgagtttttc aagcttccag tggaagaaaa gatgaaatta      300
   tactcagatg atccatcaaa gactatgaga ttatcaacaa gttttaatgt gaagaaggaa      360
   actgttcata attggagaga ttatcttaga ctgcactgtt atcctttgga caaatatgcc      420
   cctgaatggc cttctaaccc ctcttctttc agggaaatag tgagcaagta ttgcatggaa      480
   gttagacaac ttggatatag attggaagaa gccatatcag agagcctagg ccttgggaaa      540
   gattgtataa aaaatgtgtt gggtgaacaa ggtcaacata tggctatcaa tttttaccct      600
   cagtgtccac aacctgaact cacatatggc ctgccagccc atacagatcc aaatgccatt      660
   actattcttc ttcaagattt gcaagtagaa ggtctccaag ttctcaagga tggaaaatgg      720
   ttatctgtta aacctcagcc taatgccttt gtcatcaatc ttggtgatca attgcaggca      780
   ttgagtaatg gaaagtacaa aagtgtatgg catagagcta ttgtaaattc agacaaagca      840
   aggatgtctg tggcctcttt cctttgtccc tctgattgtt ccattatcag tgctccgaaa      900
   gccttaactg aagatggatc ttcagccatt tatcgagatt tcacctatac agaatattac      960
   aacaaattct ggagcaggag tttagaccag gaacgtcgtt tgaaactttt caagaaggtt     1020
   tatacatga                                                             1029
<210> 22
   <211> 1014
   <212> DNA
   <213> Nicotiana benthamiana
<220>
   <221> source
   <222> 1..1014
   <223> /mol_type="unassigned DNA" /organism="Nicotiana benthamiana"
<400> 22
   atggaagcaa aagttctttc cagcggaatc cgccactcta ctatccctca aagttacatc 60
   cgccctcaat ccgataggcc gcgcctttct gaagttgctg attgtgaaaa cgttccagta 120
   gttgatatag gttgcggtga tagaaacctt attgttcatc aaattggtga agcctgtcgt 180
   ctttatggtt ttttccaggt aattaatcat ggtgtaccaa agaatttaat agacgaaatg 240
   ctagagatag ctggggaatt ttttaggctt ccagttgaag agaagttgaa attgtactca 300
   gatgacccat cgaagacgat gagattgtcg actagtttta atgtgaaaaa ggagaaggtt 360
   cacaattgga gagattatct cagacttcat tgttatcctc ttgaaaatta cgctcctgaa 420
   tggccttcca atccttcctc tttcagggaa atcgtgagca gatattgcat ggaagttcga 480
   caactcgggt tcagattgca ggaagccata gcagagagcc taggcttaga gaaagagtgt 540
   ataaaggatg tattgggcga acaaggtcaa cacatggcta tcaatttcta tcctccttgt 600
   ccacaaccag aactcactta tgggctgcca gcacatactg atccaaatgc ccttacaatt 660
   cttcttcaag acttagaagt agctggtctt caagttctta aagatggcga atggttggcc 720
   gtcaagcctc aaccagatgc ctttgtcatt aatcttggtg atcaactgca ggcagtgagt 780
   aatgggagat acaaaagcgt atggcatcga gctattgtaa attcagacaa agccaggttg 840
   tcagtggctt cgttcctttg tccgtgcgat agcgcgaaaa tcagtgctcc aaagctcctc 900
   actgaagatg gatctcctgt catttatcag gactttacct atgctgagta ttacaaaaag 960
   ttctggagca ggaatttgga ccaggaacat tgtttggaac ttttcaagaa ctaa 1014
<210> 23
   <211> 1014
   <212> DNA
   <213> Nicotiana tabacum
<220>
   <221> source
   <222> 1..1014
   <223> /mol_type="unassigned DNA" /organism="Nicotiana tabacum"
<400> 23
   atggaagcaa aagttctttc cagcggaatc cgccactcta ctatccctca aagttacatc      60
   cgccctcaat ccgataggcc gcgcctttct gaagttgctg attgtgaaaa cgttccagta     120
   gttgatatag gttgcggtga tagaaacctt attgttcatc aaattggtga agcctgtcgt     180
   ctttatggtt ttttccaggt aattaatcat ggtgtaccaa agaatttaat agacgaaatg     240
   ctagagatag ctggggaatt ttttaggctt ccagttgaag agaagttgaa attgtactca     300
   gatgacccat cgaagacgat gagattgtcg actagtttta atgtgaaaaa ggagaaggtt     360
   cacaattgga gagattatct cagacttcat tgttatcctc ttgaaaatta cgctcctgaa     420
   tggccttcca atccttcctc tttcagggaa atcgtgagca gatattgcat ggaagttcga     480
   caactcgggt tcagattgca ggaagccata gcagagagcc taggcttaga gaaagagtgt     540
   ataaaggatg tattgggcga acaaggtcaa cacatggcta tcaatttcta tcctccttgt     600
   ccacaaccag aactcactta tgggctgcca gcacatactg atccaaatgc ccttacaatt     660
   cttcttcaag acttagaagt agctggtctt caagttctta aagatggcga atggttggcc     720
   gtcaagcctc aaccagatgc ctttgtcatt aatcttggtg atcaactgca ggcagtgagt     780
   aatgggagat acaaaagcgt atggcatcga gctattgtaa attcagacaa agccaggttg     840
   tcagtggctt cgttcctttg tccgtgcgat agcgcgaaaa tcagtgctcc aaagctcctc     900
   actgaagatg gatctcctgt catttatcag gactttacct atgctgagta ttacaaaaag     960
   ttctggagca ggaatttgga ccaggaacat tgtttggaac ttttcaagaa ctaa          1014